# EUROPEAN PATENT APPLICATION

(11) **EP 3 881 832 A1**
(43) Date of publication of application: **22.09.2021**
(21) Application number: 19900854.1
(22) Date of filing: 25.11.2019
(51) Int. Cl.: A61K 9/20, A61K 9/50, A61K 31/18

(54) **PHARMACEUTICAL COMPOSITION CONTAINING TAMSULOSIN HYDROCHLORIDE WITH EXCELLENT ACID RESISTANCE AND PREPARATION METHOD THEREFOR**

(30) Priority: 21.12.2018 KR 20180167767
(71) Applicant: Hanmi Pharm. Co., Ltd., Hwaseong-si, Gyeonggi-do 18536 (KR)
(72) Inventor: HA, Eun Ho, Hwaseong-si, Gyeonggi-do 18598 (KR); LEE, Seung Jun, Incheon 21982 (KR); IM, Ho Taek, Yongin-si, Gyeonggi-do 16909 (KR); KIM, Yong Il, Gwacheon-si, Gyeonggi-do 13835 (KR)
(74) Representative: Scholz, Volker
(86) International application number: PCT/KR2019/016280
(87) International publication number: WO 2020/130385

(57) **Abstract**

Disclosed are an acid-resistant pharmaceutical composition including tamsulosin hydrochloride, and a method of preparing the same. The pharmaceutical composition is formulated into tablets by preparing core beads including tamsulosin hydrochloride as an active ingredient, coating the core beads with an entering coating solution including a specific amount of plasticizer, adding a post mixture portion including a buffer to adjust density and a lubricant to the enteric-coated core beads, and performing tableting. The enteric coating layer is not broken during the tableting process. Thus, the acid resistance of the pharmaceutical composition is maintained. The pharmaceutical composition can be easily formulated into tablets, ensures good uniformity of dosage unit, lowers dissolution rate of tamsulosin hydrochloride, has good acid resistance, and is stable not to exhibit signification decomposition of tamsulosin hydrochloride.

## Description

### Technical Field

The present invention relates to a pharmaceutical composition containing tamsulosin hydrochloride with excellent acid resistance and preparation method therefor.

### Background Art

Tamsulosin hydrochloride is a medication that selectively acts on the genitourinary system by selectively blocking an α-adrenoceptor. Specifically, it improves the excretion rate of urine by relaxing the smooth muscle surrounding the bladder and the prostate and treats symptomatic benign prostatic hyperplasia. It is known that tamsulosin exhibits good efficacy and few side effects.

The bioavailability of tamsulosin hydrochloride is 90% or more so that it is well absorbed in the body. The half-life of tamsulosin is 9 to 13 hours in the bodies of healthy people while it is 14 to 15 hours in the bodies of benign prostatic hyperplasia patients. Accordingly, tamsulosin hydrochloride does not need to be provided as sustained sustained-release preparations for more than 12 hours or 24 hours. When it is prepared to be slowly released for about 6 hours, the drug can be maintained at a sufficient concentration for 24 hours.

Tamsulosin hydrochloride is white crystalline powder that is decomposed at about 230°C and has a physicochemical property of dissolving a little in water. Since tamsulosin hydrochloride is not a substance that is scarcely dissolved in water, the release control thereof needs to be cautiously considered.

Patent Document 1 discloses a pharmaceutical preparation for oral including tamsulosin hydrochloride-contained sustained-release granules, in which the formulation exhibits low release variation in effective ingredients because of its high sphericity and improved medication compliance because of its small size. The preparation disclosed in Patent Document 1 includes tamsulosin hydrochloride-contained sustained-release granules including tamsulosin hydrochloride, polyvinyl acetate(PVAc), hydroxypropyl methylcellulose (HPMC), and a diluent. The preparation disclosed in Patent Document 1 has the advantage of increasing the sphericity of granules through the control of a mass ratio of granules to effective ingredients and the control of the contents of PVAc and HPMC and the advantage of reducing dissolution variation to obtain stable efficacy. The preparation disclosed in Patent Document 1 is proven to exhibit a dissolution rate of tamsulosin hydrochloride of around 20% (i.e., ranging from 16.3% to 27.1%) for 2 hours in a pH 1.2 water-based buffer solution.

Patent Document 2 discloses a sustained-release pellet containing tamsulosin hydrochloride, the pellet including the same ingredients as the preparation of Patent Document 1. That is, an oral pharmaceutical formulation preparation including a tamsulosin hydrochloride-contained sustained-release pellet containing tamsulosin hydrochloride, polyvinyl acetate (PVAc), hydroxypropylmethylcellulose (HPMC), and a diluent. Patent Document 2 provides the pharmaceutical preparation having a reasonable particle size ratio of pellets and exhibiting low variation in dissolution rate. The preparation disclosed in Patent Document 2 exhibits dissolution rates of 16.7% to 31.1% (see the examples in the literature) for 2 hours in a pH 1.2 water-based buffer solution.

### Documents of Related Art

### Patent Document

Korea Patent Publication No. 2017-0073580 (June 28, 2017)
Korea Patent Publication No. 2018-0008339 (January 24, 2018)

### Disclosure

### Technical Problem

The inventors of the present patent application continuously conducted research and study on an improved acid-resistant pharmaceutical preparation of tamsulosin hydrochloride compared to the existing preparations disclosed in the patent documents of the related art and confirmed that when an enteric coating layer comprises a plasticizer in a specific amount and a buffer is added to a post mixture used in a tableting process, it is possible to prevent the enteric coating layer from being damaged in the tableting process and to improve the physical properties of the pharmaceutical preparation.

Objectives of the present invention are to provide a pharmaceutical composition of tamsulosin hydrochloride and having excellent acid resistance and to provide a method of preparing the same.

Another objective of the present invention is to provide oral formulation of tamsulosin hydrochloride.

### Technical Solution

To achieve the above objectives, the present invention provides a pharmaceutical composition of tamsulosin hydrochloride, the composition comprising tamsulosin hydrochloride as an active ingredient, an enteric-coating material, and a plasticizer. When the content of the plasticizer is adjusted to an appropriate percentage, an enteric coating is not broken so that acid resistance is maintained.

One aspect of the present invention provides a pharmaceutical composition comprising tamsulosin hydrochloride as an active ingredient and an enteric polymer and a plasticizer as other ingredients, in which the plasticizer comprising a content of 0.10% by weight to 3.5% by weight based on the total amount of the pharmaceutical composition.

The pharmaceutical composition may include an enteric coating layer formed on a core bead comprising tamsulosin hydrochloride.

The core bead may be a sustained-release core bead coated with a sustained-release coating layer including a sustained-releasing agent.

The pharmaceutical composition may include a sustained-release core bead coated with an enteric coating layer and a post mixture and may be formulated as an oral formation.

The post mixture may include a disintegrant and a buffer.

Another aspect of this invention provides:
preparing a core bead including tamsulosin hydrochloride;
coating the surface of the core bead with a coating solution including a sustained-releasing agent to prepare a sustained-release core bead;
coating the sustained-release core bead with an enteric coating solution including an enteric polymer and a plasticizer to prepare an enteric-coated sustained-release core bead;
preparing a post mixture including a disintegrant and a buffer; and
tableting by mixing the enteric-coated sustained-release core bead, the post mixture, and a lubricant. A method of preparing a pharmaceutical composition including tamsulosin hydrochloride, the method including

### Advantageous Effects

The pharmaceutical composition according to the present invention has lower friability, better dosage uniformity, lower tamsulosin hydrochloride dissolution rate in a pH 1.2 buffer solution, which contributes to better acid resistance, than conventional pharmaceutical compositions including tamsulosin hydrochloride. In addition, with the use of the pharmaceutical composition of the present invention, it is possible to prepare a tamsulosin hydrochloride-contained pharmaceutical composition that is stable because there is no significant decomposition of tamsulosin hydrochloride. This increases bioavailability of tamsulosin hydrochloride by increasing the stability of tamsulosin hydrochloride, inhibiting dissolution of tamsulosin hydrochloride in the stomach, and increasing the absorption rate of tamsulosin hydrochloride in the intestine.

### Description of Drawings

FIG. 1 is a graph showing change in dissolution rate with time for each of the tablets prepared according to Examples 1 to 3 and Comparison examples 1 and 2.
FIG. 2 is a graph showing changes in the total content of impurity in each of the tablets prepared according to Examples 1 to 3 and Comparative Examples 2, measured at an initial stage, after undergoing one-week stress conditions, and after undergoing two-week stress conditions.
FIG. 3 is a graph showing changes in the content of impurity A in each of the tablets prepared according to Examples 1 to 3 and Comparative Examples 2, measured at an initial stage, after undergoing one-week stress conditions, and after undergoing two-week stress conditions.
FIG. 4 is a graph showing changes in dissolution rate with time for each of the tablets prepared according to Examples 4 to 6 and Comparison Examples 3 and 4.
FIG. 5 is a graph showing changes in dissolution rate with time of each of the tablets prepared according to Examples 7 to 10.

### Best Mode

All technical terms used in the description of the present invention, unless otherwise defined, are used in the same sense as those understood by the ordinarily skilled in the related art.

### Pharmaceutical Composition

A pharmaceutical composition according to the present invention includes tamsulosin hydrochloride as an active ingredient and further contains enteric polymers and plasticizers.

The pharmaceutical composition according to the present invention includes tamsulosin hydrochloride as an active ingredient. The tamsulosin hydrochloride is a medication that selectively acts on the genitourinary system and is available in a variety of formulations.

In particular, the pharmaceutical composition according to the present invention may be prepared as oral formulations. The oral formulations may include capsule, tablet, dry syrup, syrup, jelly, and granule. For example, the oral formulation may be a tablet, more specifically, oral disintegrating tablet (ODT).

The tablet may be prepared through tableting. The tableting is a process of binding particles by generating a binding force between the particles with the use of a tablet press. Through the tableting, it is possible to prepare high-quality tablets having high-dosage uniformity and high-hardness uniformity.

The tablet press is a machine that is composed of various shapes of punches and dies with which powdery particles or granules are strongly pressed to form various shapes of tablet. Depending on the shapes and sizes of the punches and dies, about 50 mg to about 1500 mg-dosage tablets can be produced.

To obtain high-quality tablets, various process parameters such as the tableting pressure of the tablet press as well as the composition to form the tablets must be considered. When preparing tablets from the pharmaceutical composition according to the present invention, enteric-coated core beads, a post mixture, and additives are mixed, and a predetermined tableting pressure is applied to the obtained mixture.

Tamsulosin hydrochloride is required to act on the intestine. Therefore, since a pharmaceutical composition including tamsulosin hydrochloride rapidly melts upon reaching the intestine, the composition needs to be processed to be slowly released. However, when the pharmaceutical composition including tamsulosin hydrochloride is not acid-resistant, the drug (i.e., tamsulosin hydrochloride) is rapidly released as soon as the pharmaceutical composition reaches the stomach. As a result, it is difficult to release the effective dose of the medication in the intestine. The present invention suggests ways to improve the acid resistance of tablets comprising tamsulosin hydrochloride.

According to one specific embodiment of the present invention, a pharmaceutical composition includes tamsulosin hydrochloride as an active ingredient and an enteric polymer and a plasticizer as other ingredients. The content of the plasticizer is 0.10% by weight to 3.5% by weight based on the total amount of the pharmaceutical composition. Preferably, the content of the plasticizer is 0.1% by weight to 3.0% by weight. More preferably, the content of the plasticizer is 0.1% by weight to 2.5% by weight.

Specifically, the pharmaceutical composition according to the present invention includes a core bead comprising tamsulosin hydrochloride and an enteric coating layer surrounding the surface of the core bead. These are mixed with a post mixture to provide a form in which a plurality of microspheres is compressed.

In the case of the pharmaceutical composition, there is a possibility that the enteric coating layer is damaged due to the tableting pressure or frictional force during the tableting process and thus the acid resistance is reduced. Moreover, it is difficult to obtain homogeneous tablets because of the difference in density between the core bead and the post mixture. In addition, when there is a large difference in density between the composition of the core bead and the composition of the post mixture, there is a problem in that the edge of the tablet produced through the tableting breaks or cracks, resulting in variation in a content ratio of ingredients. That is, the defect rate of the product increases.

According to the present invention, a plasticizer is added to an enteric coating layer and the composition of the post mixture is controlled to control the densities of the core bead and the post mixture. This prevents the enteric coating layer from being damaged during the tableting process, thereby preventing deterioration of acid resistance of the pharmaceutical composition. In addition, the problems of the inhomogeneity and the high defect rate of products can be solved.

Herein after, the pharmaceutical composition of the present invention will be described in more detail.

### (1) Core Bead

In the present invention, the core bead comprises tamsulosin hydrochloride that is as an active ingredient of the pharmaceutical composition according to the present invention.

The core bead is prepared to comprise tamsulosin hydrochloride that is an active ingredient or prepared to comprise an active ingredient as well as a pharmaceutically acceptable inert seed.

The content of tamsulosin hydrochloride is 0.01% to 20% by weight based on the total amount of the pharmaceutical composition. It is preferably 0.05% to 10% by weight, and it is more preferably 0.1% to 5% by weight. Most preferably, it is 0.1% to 1% by weight. The mentioned content range is the range of content with which sufficient efficacy can be expected after a dosage form (for example, tablet) of the pharmaceutical composition is being administered.

The core beads used in the present invention can be implemented in various forms. Although the core beads are not limited to have a specific shape, size or size deviation but preferably have a substantially spherical shape to allow for the enteric coating layer or the sustained-release coating layer to be evenly formed over the entire surface of the core beads. To this end, the core beads are prepared by fluid-bed drying using GPCG-1 that is a fluid bed dryer, wet or dry extrusion, spheronization, or granulation.

To prepare the core beads, in addition to tamsulosin hydrochloride that is an active ingredient, an additional binder that are pharmaceutical acceptable may be used.

The binder is an ingredient that facilitates the binding between the components of the composition, thereby facilitating the shaping of the core beads. The binder used in the present invention is not specifically limited to a certain ingredient, and any ingredient that is commonly used in the related art may be used. Examples of the binder include hydroxypropylmethylcellulose (HPMC), carboxymethylcellulose, ethylcellulose, methylcellulose, hydroxyethylcellulose, ethylhydroxyethylcellulose, hydroxypropylcellulose (HPC), low substituted HPC (L-HPC), polyvinyl pyrrolidone, polyvinyl alcohol, polymers of acrylic acid and salts thereof, vinylpyrrolidone-vinyl acetate copolymer (e.g., Kollidon), gelatin, guar gum, partially hydrolyzed starch, alginate, xanthan or mixtures thereof. Specifically, hydroxypropylmethylcellulose (HPMC) may be used in the present invention.

In the pharmaceutical composition of the present invention, the binder may be included preferably in a content of 0.01% to 10% by weight, more preferably 0.05% to 5% by weight, and most preferably 0.1% to 1% by weight, based on the total amount of the pharmaceutical composition. When the content of the binder is in that range, the core beads are easy to prepare and can be formulated in the desired dosage form.

On the other hand, when preparing the core beads including an inert seed, the core beads are prepared in the form in which the active ingredient and the inert seed are mixed, the form in which the active ingredient is embedded in the inert seed, the form in which the active ingredient is coated on the surface of the inert seed, or any of other possible forms. In one embodiment of the present invention, the active ingredient is coated on the surface of the inert seed.

An inert core consists of inert materials and serves as the core of the formulation of the present invention. The core may be made from one or more selected from sugar sphere, starch, mannitol, sucrose, microcrystalline cellulose, and mixtures thereof. The size of the inert core is preferably in a range of 100 µm to 1500 µm in terms of the productivity and yield of the fluid bed assembling process. In the case of the form in which the active ingredient is embedded in the inert seed, the inert core may be implemented as a porous particle.

In the case of the form in which the active ingredient is coated on the surface of the inert seed, a coating solution including the active ingredient is prepared and the coating solution is applied to the surface of the inert seed by a coater, a fluidized bed system, a fluidized bed processor, a fluid bed granulator, or the like. More particularly, the coating may be carried out with a fluidized bed system, a centrifugal granulator, or Granurex™ (manufactured by Freund). Preferably, when the coating is performed with the fluidized bed system, top spray coating, bottom spray coating, or tangential spray coating is used.

The core bead used in the present invention may have a sustained-release coating layer on the surface thereof.

The sustained-release coating layer allows a medication to be slowly released to prevent side effects that may occur due to a rapid increase in the concentration of the medication in the blood when the medication is rapidly released.

The sustained-release coating layer is formed by coating a sustained-releasing agent. The sustained-releasing agent not only serves to support a pharmaceutical formulation but also plays an important role in forming pores in the formulation in water after a certain period of time. The sustained-releasing agent used in the present invention is not specifically limited to a certain substance, and any composition that is commonly used in the related art may be used.

The sustained-releasing agent is at least one or more material selected from the group consisting of cellulose derivatives, gums, hydrophilic or hydrophobic (meth)acrylate copolymers, hydrophilic or hydrophobic polyvinyl derivatives, polyethylene derivatives, carboxyvinyl compounds, and polysaccharides

Specifically, the sustained-releasing agent includes at least one or more compound selected from the group consisting of hydroxypropylmethylcellulose, hydroxypropylcellulose, hydroxymethylcellulose, hydroxyethylcellulose, methylcellulose, ethylcellulose, sodiumcarboxymethylcellulose, cellulose acetate, cellulose acetate phthalate, methylhydroxyethylcellulose, guar gum, locust bean gum, tragacanth gum, carrageenan, acacia gum, xanthan gum, arabic gum, gellan gum, karaya gum, tara gum, tamarind gum, gati gum, polyvinyl alcohol, polyvinylpyrrolidone, polyvinyl acetyldiethylaminoacetate, polyethylene derivatives, polyethylene glycol, polyethylene oxide, carbomer, dextrin, polydextrin, dextran, pectin, pectin derivative, alginate, polygalacturonic acid, xylan, arabinoxylan, arabinogalactan, starch, hydroxypropyl starch, amylose, amylopectin, fluran, mannan, purcelleran, carrageenan, glucosamine, chitosan, chitin, gelatin, collagen, casein, agar, albumin, poly(methacrylic acid-methyl methacrylate) copolymers, poly(methacrylic acid-ethyl methacrylate) copolymers, polyvinyl acetate, poly(ethyl acrylic acid-methyl methacrylate) copolymers, poly(ethyl acrylic acid-methyl methacrylate-trimethylaminoethyl methacrylate) copolymers.

One specific example of the sustained-releasing agent is at least one selected from the group consisting of polyvinyl acetate and mixtures thereof, ethylcellulose, hydroxypropylmethylcellulose (HPMC), and mixtures thereof. When the sustained-releasing agent is included, the drug has constant releasing behavior with pH-independent, thus, can achieved sustained released, as desired.

The most preferably sustained-releasing agent in the present invention is polyvinyl acetate or a certain mixture including polyvinyl acetate. The polyvinyl acetate is most useful for the formulation of the present invention because it has constant releasing behavior with pH-independent and allows for long lasting release over time when it is left underwater. The polyvinyl acetate may have a weight average molecular weight in a range of 100,000 to 500,000 but is not limited thereto. In the present invention, as the sustained-releasing agent polyvinyl acetate is implemented as a diluted aqueous solution or a powder, either of which includes only polyvinyl acetate or a mixture of polyvinyl acetate.

For example, the sustained-releasing agent used in the present invention may be a mixture including polyvinyl acetate and polyvinylpyrrolidone, or a mixture including polyvinyl acetate and being stabilized with polyvinylpyrrolidone and sodium laurylsulphate. Specifically, Kollidon SR™(BASF) or Kollicoat SR30D is used as the sustained-releasing agent.

In the pharmaceutical composition of the present invention, the sustained-releasing agent is included preferably in a content of 0.1% by weight to 15% by weight, more preferably in a content of 0.5% by weight to 10% by weight, and most preferably in a content of 1% by weight to 5% by weight, based on the total amount of the pharmaceutical composition. When the content of each component is below the range described above, the effect expected for the sustained-releasing agent cannot be obtained. On the other hand, when the content of each component exceeds the range described above, the release of a medication will not be easily controlled. Therefore, the content of each component of the pharmaceutical composition must be suitably controlled within the range described above.

The sustained-release coating layer is formed by: preparing a coating solution composed of a sustained-releasing agent and a solvent selected from the group consisting of purified water, ethanol, acetone, aqueous solution of ethanol, aqueous solution of acetone, and a mixture of ethanol and acetone; and applying the coating solution using a coater, a fluidized bed system, a fluidized bed processor, or a fluidized bed granulator. More specifically, a fluidized system with bottom spray, a centrifugal granulator, or GRANUREX® (manufactured by Freund corporation) is be used.

In addition to the composition, the core beads used in the present invention additionally include a variety of biologically inactive ingredients for various supplemental purposes such as coating efficiency, stability of active ingredients, appearance, color, protection, maintenance, binding, performance improvement, manufacturing process improvement, or auxiliary release control.

The biologically inactive ingredients may be added when preparing the cores and will have little or no effect on the release of the active ingredients. For example, the additional inactive ingredients include diluents, sugars, sugar alcohols, polymers, colorants, flavoring agents, sweeteners, surfactants, lubricants, stabilizers, antioxidants, foaming agents, paraffin, or waxes. One or more additive selected from the examples may be added. The biologically inactive ingredient may be mixed, for use, with at least one or more selected from the group consisting of tamsulosin hydrochloride, inert seed, or a sustained-releasing agent.

For example, the biologically inactive ingredient may be a diluent.

The diluent refers to a material that allows granules to remain in their shape and do not melt in the body. As the diluent, at least one or more selected from the group consisting of microcrystalline cellulose (MCC), talc, lactose, and inorganic carriers such as dibasic calcium phosphate, dibasic calcium phosphate dihydrate, tribasic calcium phosphate, and combinations thereof, but the diluent is not limited thereto. According to one embodiment, the diluent is selected from the group consisting of microcrystalline cellulose, talc or a combination thereof.

The core beads are made from a mixture of the active ingredient, the inert seeds, and the sustained-releasing agent are prepared in various forms.

In one embodiment of the present invention, the core beads are spherical core beads spherionized including tamsulosin hydrochloride, a binder, and inert seeds.

In other embodiment of the present invention, the core beads are sustained-release core beads prepared by coating a coating solution comprising tamsulosin hydrochloride and binder on inert seed and then additionally coating them with coating solution including sustained-releasing agent.

In another embodiment of the present invention, the core beads are sustained-release core beads prepared by coating a coating solution comprising tamsulosin hydrochloride, binder and lubricant on inert seed and then additionally coating them with coating solution including sustained-releasing agent and lubricant.

In addition to the above-described forms, selection of the specific materials constituting the core bead, the use of the materials, and the method of adding the specific materials to the core of the present invention are considered to be easily performed based on the skill level of those ordinarily skilled in the art, and they can be modified without departing from the scope of the invention.

### (2) Enteric Coating Layer

The enteric coating layer refers to a later coated on the surface of the core bead so that the core bead can endure while passing through the stomach. The enteric coating layer functions to control the release of tamsulosin hydrochloride in the stomach with low pH such that the release is carried out at or below a specific level. This results in prevention of the side effects of tamsulosin hydrochloride in the gastrointestinal tract and facilitate the release of tamsulosin hydrochloride in the intestine. Therefore, the bioavailability of tamsulosin hydrochloride is improved.

The enteric coating layer is formed from enteric polymers that are commonly used in the technical field to which the present invention pertains. The enteric polymer is a pH-dependent polymer. The enteric polymer is stable under an acidic condition which is under pH 5, and is dissolved under a weak acidic condition meaning pH 5 or above or a neutral condition.

The enteric polymer used in the present invention is not limited to a specific polymer, but any enteric polymer that is commonly used in the field of the invention can be used. Specifically, for example, the enteric polymer is selected from the group consisting of hydroxypropylmethylcellulose phthalate, hydroxypropylmethylcellulose acetate succinate, polyvinyl acetate phthalate, cellulose acetate phthalate, cellulose acetate trimellitate, shellac, polymethacrylate copolymers (including, methacrylic acid-methyl methacrylate copolymers, and methacrylic acid-ethyl acrylate copolymers), and any combination thereof.

Preferably, the enteric polymer comprises one or more kinds of acid-resistant acrylic polymers selected from the group consisting of methacrylic acid-methyl methacrylate copolymers and methacrylic acid-ethyl acrylate copolymers.

The acid-resistant acrylic polymer is an enteric polymer that is pH-dependent. Thus, it does not dissolve under strong acidic conditions (about pH 4.5 or under) but dissolves at or over pH 5.5. Typically, a methacrylic acid-methylmethacrylate copolymer or a methacrylic acid-ethyl acrylate copolymer is used as the enteric polymer. Specifically, a material selected from the group consisting of Eudragit-L30 D-55, Eudragit-NE30D, Eudragit-LlOO, Eudragit-LlOO D-55, Eudragit-L12,5, Eudragit-S100, Eudragit-S12,5, Eudragit-FS 30 D that are all brand names and any combination thereof can be used. Preferably, Eudragit-L30 D-55 or Eudragit-NE30D may be used. More preferably, Eudragit-L30 D-55 and Eudragit-NE30D may be used in a mixed form.

Eudragit-L30 D-55 is a methacrylic acid-ethylacrylate copolymer (1:1) aqueous dispersion. It hardly dissolves in a low pH (for example, pH 5.5 or lower) condition, but begins to dissolve when the pH becomes 5.5 or higher. Due to this characteristic, it is widely used as an enteric polymer. Eudragit-L30 D-55 can be used as a 30% (m/V) aqueous dispersant of acrylate polymer including sodium lauryl sulfate and polysorbate 80 as emulsifiers.

Eudragit-NE 30D is an aqueous dispersion of ethyl acetate and methyl methacrylate copolymer.

The enteric polymer is included preferably in a content of 0.1% to 20% by weight, more preferably in a content of 0.5% to 10% by weight, and most preferably in a content of 1% to 5% by weight, based on the total amount of the pharmaceutical composition of the present invention. When the content of the enteric polymer is within those ranges, the function of the entering coated layer can be achieved. That is, the release of drugs within the gastrointestinal tract can be suppressed. When the content of the enteric polymer is below the lower limit of the range, drugs may release in the gastrointestinal tract due to the low acid resistance. Conversely, when the content of the enteric polymer is higher than the upper limit of the range, it is difficult to prepare the desired dosage form (for example, tablet) because the viscosity of the pharmaceutical composition is increased and is thus difficult to be coated.

As such, the enteric polymer can increase the acid resistance of the dosage form (formulation), but when the pharmaceutical composition is prepared into tablets, damage to the enteric coating layer made of the enteric polymer may occur during the tableting process. As a result, since the acid resistance of the finished dosage form (i.e., tablet) is low, there is a likelihood that the active ingredient releases in the gastrointestinal tract after the tablet is administered. This results in significant deterioration of the bioavailability of the drug.

Accordingly, in the present invention, a plasticizer is used to increase the acid resistance of the pharmaceutical composition by suppressing damage to the enteric coating layer in the tableting process.

A plasticizer refers to a material that increases the free volume of a polymer to facilitate segmental motion of the polymer. The plasticizer serves to increase durability of drug by imparting flexibility and elasticity to the coating layer.

Plasticizers come in various forms such as aromatic plasticizers and aliphatic plasticizers, and are used in consideration of biocompatibility, compatibility, stability, processability, persistence, and price. Although various types of plasticizers are already known in the technical field of the present invention, but there is no example that a plasticizer is added to a pharmaceutical composition including tamsulosin hydrochloride as an active ingredient.

In the present invention, a plasticizer is added to improve the stability of tamsulosin hydrochloride and increase the acid resistance of the enteric coating layer. The enteric coating layer is formed on the surface of the core bead by preparing an enteric coating material having acid resistance and spraying the coating material with a fluidized bed system. When a plasticizer is added to the enteric coating material, the flexibility of the enteric coating layer increases so that the quality (i.e., coating film properties) of the coating layer will be improved. Therefore, a stable coating layer is formed on the surface of the core bead as an enteric coating layer that is not easily torn or peeled off by an external force (e.g., pressure during tableting), thereby increasing the acid resistance of the formulation as well as increasing the stability of tamsulosin hydrochloride.

According to Experimental Example 1, tablets of tamsulosin hydrochloride were prepared, in which citrate was used as a plasticizer in a content of 0.05% to 3.75% by weight based on the total weight of the composition.

In Experimental Example 1, impurities were tested. By testing the tablet of tamsulosin hydrochloride for stability, it was found that the film of an enteric coating layer was cracked depending on to the content of the plasticizer under stress conditions so that the tamsulosin hydrochloride inside the coating layer was exposed and decomposed. When the content of the plasticizer was excessively high, it was confirmed that the decomposition of tamsulosin hydrochloride was severe so as to serve as a tablet.

In addition, in a test of examining dissolution in an artificial gastric fluid, which is related to the acid resistance test of Experimental Example 1, the release rate of tamsulosin hydrochloride was changed according to the content of the plasticizer. When the content of the plasticizer was significantly low, the initial dissolution rate and the final dissolution rate of tamsulosin hydrochloride was high so that the function of the enteric coating layer was deteriorated.

As such, the content of the plasticizer has a significant effect on the physical properties of the pharmaceutical formulations. Accordingly, the present invention maximizes the stability of tamsulosin hydrochloride and the function of the enteric coating layer by limiting a content ratio of the plasticizer relative to the total pharmaceutical composition.

Specifically, according to the present invention, the plasticizer is included in a content of 0.1% by weight to 3.5% by weight based on the total amount of the pharmaceutical composition. The content of the plasticizer is set to be preferably in a range of 0.1% to 3.0% by weight, more preferably in a range of 0.1% to 2.5% by weight, and most preferably in a range of 0.15% to 2.5% by weight. Alternatively, the content of the plasticizer is set to be in a range of 0.3% to 2.5% by weight, and more preferably in a range of 0.3% to 2% by weight, based on the total amount of the pharmaceutical composition. When the plasticizer is included in an amount that falls within any one of the ranges described above, the enteric coating layer has sufficient acid resistance and stability. When the content is lower than the lower limit of that range, the acid resistance of the enteric coating layer is reduced, resulting in high initial and final release rates of the drug in the stomach. Conversely, when it exceeds the upper limit of that range, there is a problem in that tamsulosin hydrochloride is decomposed. Therefore, the content of the plasticizer needs to be suitably determined within any of the ranges described above.

In addition to the content of the plasticizer, the selection of the plasticizer is also very important.

The plasticizer is selected in consideration of compatibility with the composition of the enteric coating layer, stability, and physical properties such as processability.

The present invention uses an acid-resistant acrylic polymer as the enteric polymer. To improve the physical properties of the enteric coating layer, at least one or more material selected from the group consisting of polyethylene glycol, polypropylene glycol, polyglycolic acid, polybutylene adipate, glycerin, tributyl sebacate, triacetin, and triethyl citrate is used. Polymers such as polyethylene glycol, polypropylene glycol, polyglycolic acid, and polybutylene adipate have a characteristic that the elongation of the coating film increases, and the tensile strength of the coating film decreases as the molecular weight increases. Given that fact, a polymer having a weight average molecular weight of 1000 or less is preferable in terms of physical properties. In exemplary embodiments, when triethyl citrate is used, an enteric coating layer with a high-quality film can be formed.

The high-quality film means that an enteric coating layer is formed to have a uniform thickness on the surface of the core bead, has good physical properties such as sufficient strength, hardness, elongation, and flexibility required for a coating film, and can rapidly dissolve in intestinal fluids. The required physical properties can be satisfied by controlling the contents of the enteric polymer and the plasticizer.

Preferably, in the pharmaceutical formulation of the present invention, the enteric polymer and the plasticizer are included in a weight ratio in a range of 1:0.03 to 1:1.0, more preferably 1:0.03 to 1:0.75, and most preferably 1:0.03 to 1:0.2, but the ratio is not limited thereto. In the formulation of the invention, when the enteric polymer and the plasticizer are included in a weight ratio in any of the ranges described above, the required physical properties are satisfied. Thus, the enteric coating layer remains stable, has sufficient acid resistance, and enables tamsulosin hydrochloride to be stably retained.

This enteric-coating layer is formed by preparing a coating solution comprising an enteric polymer and coating the surface of the core bead with the coating solution.

As a coating solvent for preparation of the coating solution, purified water, ethanol, acetone, aqueous solution of ethanol, aqueous solution of acetone, or a mixture of ethanol and acetone may be used.

In addition to the composition described above, the coating solution for an enteric coating layer further includes various biologically inactive ingredients for supplemental purposes such as coating efficiency, stability of active ingredients, appearance, color, protection, maintenance, binding, performance improvement, manufacturing process improvement, or auxiliary release control.

The inactive ingredients include a diluent, sugar, sugar alcohols, polymer, colorant, flavoring agent, sweetener, surfactant, lubricant, stabilizer, antioxidant, foaming agent, preservative, disintegrant, buffer, bulking agent, diluent, paraffin, or wax. One or more inactive ingredients may be used. For example, the biologically inactive ingredient may be a diluent. The specific composition is the same as described above. A specific example of the diluent is talc.

According to one embodiment of the present invention, the enteric coating layer is formed by applying the coating solution with a coater, a fluidized bed system, a fluidized bed processor, a fluid bed granulator, or the like. More specifically, the coating may be carried out with a fluidized bed system, a centrifugal granulator, or Granurex™ (manufactured by Freund). Preferably, when the coating is performed with the fluidized bed system, top spray coating, bottom spray coating, or tangential spray coating is used.

### Oral formulation

The pharmaceutical composition according to the present invention may be prepared as dosage forms for oral administration.

The oral formulation may be used not only for treatment of any disease known as an indication for tamsulosin hydrochloride, but also for treatment of any disease that may be found as an indication of tamsulosin hydrochloride in the future. In the present specification, the term "treatment" has meaning including treatment, improvement, amelioration, and management of a disease. In one embodiment, the pharmaceutical formulation may be used for treatment of benign prostatic hyperplasia, urination disorders accompanying prostatic hyperplasia, and acute urinary retention.

An oral formulation according to the present invention may comprise 0.2 mg to 0.8 mg of tamsulosin hydrochloride per unit dosage form. Even though the oral formulation is administered once a day, the active ingredient of the formulation may exhibit an optimized dissolution pattern.

In one embodiment, the formulation comprising of tamsulosin hydrochloride may be prepared as a complex including tamsulosin hydrochloride and an additional active ingredient that can be used in combination with tamsulosin hydrochloride. The additional active ingredient may be, for example, tadalafil, dutasteride, solifenacin, or finasteride, but is not limited thereto.

Oral formulations include capsules, tablets, dry syrup formulations, syrup formulations, jelly formulations, and granules. Among these, the pharmaceutical composition according to the present invention is formulated as tablets. The tablets include general tablets commonly used in the field of the invention, double tablets, chewable tablets, and fast disintegrating tablets.

As an oral formulation, tablets have the advantages of providing an accurate dosage, being easy to handle, being easy to take, being easy to mass-produce, and being inexpensive. In addition, it is easy to correct bitter taste, odor, and irritation through the coating, and it is easy to control changes in the dissolution and absorption patterns of the drug by controlling the molding.

Tablets may vary in shape. For example, the tablets may take an ovoid, triangular, almond, peanut, parallelogram, circular, pentagonal, hexagonal, or trapezoidal shape. The desirable tablet shape is round, ovoid, or parallelogram.

Oral formulation (for example, tablets) comprising tamsulosin hydrochloride are prepared by adding a post mixture to the core beads coated with the enteric coating layer described above, and then applying a certain level of tableting pressure. As a result, the oral formulation has a form in which a plurality of microspheres is compressed after the core beads coated with the enteric coating layer is mixed with the post mixture.

The post mixture may be a composition used for conventional tablet production. The post mixture used in the present invention includes at least one or more of a disintegrant and a buffer. The disintegrant and the buffer are components required for preparation of tablets. The composition thereof is determined in consideration of the process characteristics of the oral formulation prepared through a tableting process.

The oral formation according to the present invention is prepared through tableting. During the tableting process, there is a possibility that the enteric coating layer is damaged due to the tableting pressure or frictional force and the acid resistance is deteriorated. In addition, it is difficult to obtain homogeneous tablets due to the density difference between the core bead and the post mixture. In addition, when there is a large difference in density between the core bead and the post mixture, there is a problem in that the edge of the tablet produced through the tableting breaks or cracks, resulting in variation in a content ratio of ingredients. That is, the defect rate of the product increases.

Given these problems, the composition of the post mixture is selected, and its content is limited to eliminate the deterioration of the acid resistance of oral formulations and the content ratio non-uniformity.

A disintegrant refers to a composition that can maintain an appropriate hardness of an oral formulation and can easily decompose an oral formulation to be easily absorbed in the body when the oral formulation is administered. As the disintegrant, one or more than two materials may be selected from those that are commonly used for preparation of tablets. For example, mannitol (e.g., spray-dried mannitol), sorbitol, fructose, lactose, dextrose, xylitol, glucose, lactose, white sugar, fructose, maltose, maltodextrin, erythritol, arabitol, crospovidone, croscarmellose sodium, microcrystalline cellulose (MCC), pregelatinzed starch, starch, derivatives of starch, Kollidone®, carboxymethylcellulose, F-Melt™ (manufactured by Fuji chemical), Ludiflash® (manufactured by BASF), and the like can be used as the disintegrant. Specifically, the disintegrant includes any one or more materials selected from the group consisting of mannitol, F-Melt™, sorbitol, xylitol, lactose, and pregelatinized starch. More specifically, the disintegrant is mannitol, F-Melt, sorbitol, xylitol, or a mixture thereof. The spray-dried mannitol may be prepared by spraying and drying a crystalline mannitol solution, or by purchasing a commercially available form (for example, PEARLITOL SD 200WP manufactured by Roquette in France, or PARTECK M200 manufactured by Merck in Germany)

There are two types of F-Melt: type M and type C. The F-Melt is a mixture of D-mannitol, xylitol, microcrystalline cellulose, crospovidone, magnesium aluminometalsilicate, and calcium phosphate and is commercially available.

According to a preferred embodiment of the present invention, as a disintegrant for the oral formulation of the present invention, F-melt type C, mannitol, or both may be used. For reference, the F-melt type C is comprised of 62.3% to 67.2% by weight of mannitol, 16.2% to 19.8% by weight of microcrystalline cellulose, 3.4% to 4.6% by weight of anhydrous dibasic calcium phosphate, 7.0% to 9.2% by weight of crospovidone, and 4.2% to 5.8% by weight of xylitol.

High-quality oral formulations require a certain level of an apparent density, a low friability, good content ratio uniformity, and a low dissolution rate of the active ingredient in the gastrointestinal tract. These physical properties are closely related to the composition of the post mixture.

The apparent density or filling volume is related to the fine particles which mean enteric-coated core beads, the composition of the post mixture, and the bonding between the fine particles and the post mixture. When the apparent density is equal to or higher than a predetermined level, the core beads and the post mixture can be tightly bonded to each other. As a result, the friability of the finished tablets is lowered, resulting in a decrease in the defective rate of the tablets.

Preferably, the oral formulation of the present invention has an apparent density of 0.6 mg/mL to 0.8 mg/mL, preferably 0.65 mg/mL to 0.78 mg/mL, and more preferably 0.70 to 0.75 mg/mL when the core beads are enteric-coated core beads. In addition, the apparent density of the composition of the post mixture in a range of 0.40 to 0.52 mg/mL, and preferably in a range of 0.42 to 0.49 mg/mL.

It is preferable that the apparent density ratio of the enteric-coated core bead to the composition of the post mixture is in a range of 1:0.57 to 1:0.71. Low apparent density means that the bonding force is low. In this case, since the apparent density of the composition of the post mixture is low, disintegration may occur preferentially after oral administration. With the apparent densities being in the ranges described above, tablets that are uniform in content ratio can be obtained.

For physical properties including the apparent density, the composition of the post mixture includes the disintegrant in a content of 50% to 90% by weight based on the total weight of the pharmaceutical composition. When the content of the disintegrant is lower than the range described above, the oral formulations are easily decomposed during transport, preservation, or storage. In this case, product defects occur, or rapid dissolution occurs after the drug is taken. Therefore, drug efficacy cannot be expected due to fast release of the medication. Conversely, when the content of the disintegrant is excessively high, excessively fast dissolution occurs, resulting in a high initial release rate of the active ingredient. As a result, it difficult to obtain a long-lasting effect.

The post mixture used in the invention may further comprises a buffer. According to one embodiment, colloidal silica (AEROSIL®) is used as the buffer. In addition to the colloidal silica, other buffers also can be used.

The buffer can not only lower the apparent density but also prevent abrasion, friction, and breakage of the core bead, which are related to the quality of the oral formulations, during the tableting process, thereby suppressing dissolution of the active ingredient in the gastrointestinal tract. That is, the buffer increases the acid resistance.

In addition, when the hardness of tablets is low, the tablets will easily break during the tableting process, resulting in an increase in a product defect rate. Therefore, tablets are required to have a certain degree of hardness or higher. This can be achieved by controlling the apparent density of the buffer.

In addition, the use of the buffer increases the fluidity and dispersion of particles during the tableting process, thereby allowing the enteric-coated core beads to be uniformly included in each tablet.

In particular, the use of the buffer increases the acid resistance of oral formulations. According to Experimental Example 2 of the present invention, when a disintegrant and a buffer were used in combination as the post mixture, the dissolution rate was 20% or lower in gastric fluid after 2 hours. However, when the content of the buffer is significantly low or excessively high, the initial and final dissolution rates increased. That is, without the buffer, the bioavailability of tamsulosin hydrochloride of an oral formulation was reduced.

Preferably, the content of the buffer included in the post mixture used in the present invention is determined in consideration of the aforementioned physical properties.

Specifically, the content of the buffer is set to be in a range of 0.5% by weight to 3.0% by weight and preferably in a range of 0.5% to 2.5% by weight with respect to the amount of the pharmaceutical composition of the present invention. When the content is determined to be within the range, high content uniformity and acid resistance can be obtained. When the content of the buffer is below the range, the effects described above cannot be achieved. Conversely, when the content exceeds the range, the apparent density will decrease, the friability will increase, and the content uniformity and acid resistance of the oral formulation will decrease.

According to one embodiment of the present invention, an oral formulation includes a core bead comprising tamsulosin hydrochloride as an active ingredient, an enteric coating layer including an enteric polymer and a plasticizer, and a post mixture including a buffer, a disintegrant, or both. The plasticizer is included in a content of 0.10% to 3.5% by weight with respect to the total amount of a pharmaceutical composition. The enteric-coated core bead included in the oral formulation has an apparent density in a range of 0.6 to 0.8 mg/mL, and the composition of the post mixture has an apparent density in a range of 0.40 to 0.52 mg/mL.

In addition to the composition, the oral formulation of the present invention additionally includes a variety of biologically inactive ingredients for various supplemental purposes such as coating efficiency, stability of the active ingredient, appearance, color, protection, maintenance, binding, performance improvement, manufacturing process improvement, or auxiliary release control.

The inactive ingredients include a diluent, sugar, sugar alcohols, polymer, colorant, flavoring agent, sweetener, surfactant, lubricant, stabilizer, antioxidant, foaming agent, preservative, disintegrant, buffer, bulking agent, diluent, paraffin, and wax. Among them, one or more inactive ingredients may be used. For example, the biologically inactive ingredient may be a lubricant.

The lubricant exhibits glidant or antitacking effects so that tablets can be easily released from a compression die, problems such as adhesion can be eliminated during the tableting process, and cracking or delamination of tablets can be prevented during tableting. As the lubricant, one or more materials selected from the group consisting of stearic acid, glyceryl behenate, glyceryl mono stearate, magnesium stearate, calcium stearate, sodium stearyl fumarate, silicon dioxide, talc, and magnesium silicate. Preferably, magnesium stearate is used as the lubricant.

The content of the lubricant is 10% by weight or less, 0.1% to 10% by weight, or 0.5% to 5% by weight, with respect to the total pharmaceutical composition. More preferably, the lubricant is stearate magnesium, and the content thereof is less than 3% by weight, preferably 0.5% to 2.5% by weight, and most preferably 1.0% to 2.0% by weight with respect to the pharmaceutical composition. Specifically, the use of the lubricant offers the advantage of preventing sticking during the tableting process, thereby lowering the defect rate. However, the excessive use of the lubricant makes it difficult to satisfy the dissolution rate standard of medications. Therefore, the content of the lubricant is preferably determined within the specified range. The oral formulation having the composition according to the present invention has a dissolution rate of 20% or less after 2 hours when the dissolution rate is measured with a pH 1.2 buffer solution of 500 mL and a rotational speed of 75 rpm according to the paddle method, which is the second dissolution test according to the United States Pharmacopoeia (USP). Therefore, it is possible to minimize dissolution in the stomach and maximize dissolution in the intestine.

In addition, as a result of measuring for 4 minutes at 25 rpm with a friability measuring device (device name: TAR200, manufacturer: ERWEKA), the friability was 0.5% or less. That is, the quality of the tablet was high.

In addition, the oral formulation according to the present invention satisfies a uniformity level of 15 that is a standard of uniformity of dosage unit for a tamsulosin hydrochloride tablet, which is specified in the United States Pharmacopoeia (USP).

In addition, there is an advantage of excellent storage stability of the tablet because the decomposition rate of tamsulosin hydrochloride is low under stress conditions (60°C).

### Preparation Method

The method of preparing the oral formulation according to the present invention is not particularly limited in the present invention, and the oral formulation according to the present invention can be formulated by any method known in the art.

According to one embodiment of the present invention, an oral formulation is prepared by:
a) providing core beads;
b) preparing a post mixture including a disintegrant and a buffer; and
c) after mixing the core beads, the post mixture, and an additive and tableting the obtained mixture.

First, the core beads used in step a) are various shapes of core beads described above.

According to another embodiment of the present invention, a pharmaceutical composition comprising tamsulosin hydrochloride is prepared by:
preparing core beads including tamsulosin hydrochloride;
coating the surface of the core beads with a coating solution including an extended releasing agent to prepare sustained-release core beads;
coating the sustained-release core beads with an enteric coating solution including an enteric polymer and a plasticizer to prepare enteric-coated sustained-release core beads having an enteric coating layer;
preparing a post mixture including a disintegrant and a buffer; and
after mixing the enteric-coated sustained-release core beads, the post mixture, and a lubricant, and tableting the obtained mixture.

The enteric coating layer includes an acid-resistant acrylic copolymer and a triethyl citrate as plasticizer. In addition, the sustained-release core bead is a core bead coated with Kollicoat SR30D, which is a sustained-releasing agent. The inside of the sustained-release core bead is provided with a microcrystalline cellulose inert seed that is coated with a mixture of tamsulosin hydrochloride, a binder, and a diluent.

Specifically, the enteric-coated sustained-release core beads are prepared by:
preparing core beads including tamsulosin hydrochloride, the binder, and the diluent;
coating the core beads with a coating solution comprising a sustained-releasing agent to produce the sustained-release core beads coated with the sustained-release coating layer; and
coating the sustained-release core beads with an enteric coating solution including an enteric polymer and a plasticizer to prepare the enteric-coated sustained-release core beads having the enteric coating layer.

The post mixture in step b) has the composition described above.

The additive in step c) is a lubricant, and the tableting is performed with an existing tableting machine.

The tableting is not specifically limited in the present invention. Direct powder compression or granule compression may be used in the tableting process. Tablets may be prepared using various tableting machines that are respectively suitable for the tableting methods. When using the direct powder compression method, the manufacturing process time is shortened by half, and the contamination of the work environment due to a pigment originating in an active ingredient is reduced. In addition, when tableting is performed through the wet granulation and active ingredient mixing, there is an advantage that the size of tablets can be reduced.

### Mode for Carrying out the Invention

### [Example]

Hereinbelow, the present invention will be described in more detail with reference to examples. However, the examples described below are provided only to aid understanding of the present invention and thus should not be construed to limit the scope of the present invention.

### Preparation Example 1 Preparation of Sustained-release Core Bead

Core beads comprising tamsulosin hydrochloride and coated with a sustained-release coating layer were prepared.

First, tamsulosin hydrochloride, hydroxypropylmethylcellulose (HPMC) serving as a binder, ethanol, and purified water were mixed, in a content ratio shown in Table 1, in a high-speed mixer, and then talc was added to the mixture to produce a coating solution. Microcrystalline cellulose (MCC, Cellets 175, particle size distribution of 150 µm to 200 µm) were put into a fluidized bed coater as inert seeds, and coating was carried out by spraying the coating solution through a bottom spray coating process. After the spraying of the coating solution was finished, the resultant was dried to produce core beads comprising tamsulosin hydrochloride.

To prepare a sustained-release coating layer on the surface of the core bead, Kollicoat® SR 30D(EP), which is a sustained-releasing agent was dissolved in water, and talc was added thereto to produce a sustained-release coating solution. The prepared core beads were put into a fluidized bed system, and the coating was carried by spraying the sustained-release coating solution from the bottom. After the spraying of the coating solution was finished, the resultant was dried to produce sustained-release core beads coated with a sustained-release coating layer.

**[Table 1]**

| | Component | mg/T |
|---|---|---|
| Core bead | Microcrystalline cellulose (Cellet 175) | 20.00 |
| | Tamsulosin hydrochloride | 0.40 |
| | hydroxypropylmethylcellulose | 0.20 |
| | Talc | 0.20 |
| | Ethanol | (25.20) |
| | Purified water | (2.80) |
| Total of core beads | | 20.80 |
| Sustained-release coating solution | Kollicoat SR 30D | 4.49 |
| | Talc | 0.46 |
| | Purified water | (14.96) |
| Total of sustained-release core beads | | 25.75 |

### Experimental Example 1: Comparison of Physical Properties between Tablets on Content of plasticizer

Tablets were manufactured by binding enteric-coated sustained-release core beads having a sustained-release coating layer through tableting. The enteric coating layer may be damaged and thus acid resistance may be deteriorated during the tableting process. According to the present invention, the acid resistance can be increased by adding a plasticizer. In this experimental example, each tablet was manufactured by varying the content of a plasticizer, and the physical properties of the prepared tablets were compared.

### (1) Preparation of Tablet

Tablets were prepared in accordance with the composition shown in Table 2. As core beads, the sustained-release core beads prepared according to Preparation Example 1 were used.

Eudragit L30-D55 and Eudragit NE30D were used as an enteric coating material, and triethyl citrate was used as the plasticizer. Eudragit L30-D55 and Eudragit NE30D were dissolved in purified water in a mixer, talc and triethyl citrate were added thereto to prepare an enteric coating solution. In Examples 1 to 3, the contents of triethyl citrate used as the plasticizer was 0.1% by weight, 0.3% by weight, and 0.5% by weight, respectively. In Comparative Examples 1 and 2, the contents were adjusted to 0.05% by weight and 7.5% by weight, respectively.

The sustained-release core beads prepared in Preparation Example 1 were taken and put into a fluidized bed system, and coating was carried by spraying the prepared coating solution from the bottom. After the spraying of the coating solution was finished, microspheres (i.e., enteric-coated sustained-release core beads) that are the sustained-release core beads on the surface of which an enteric coating layer is formed were obtained.

Tablets were prepared by adding a post mixture including F-Melt Type C as a disintegrant, AEROSIL® as a buffer, and a lubricant and tableting the resultant.

**[Table 2]**

| Composition | | Example 1 | Example 2 | Example 3 | Comparative Example 1 | Comparative Example 2 |
|---|---|---|---|---|---|---|
| Sustained-release core beads (mg) | | 25.75 | 25.75 | 25.75 | 25.75 | 25.75 |
| Enteric coating solution | Eudragit L30-D55 | 3.40 | 3.40 | 3.40 | 3.40 | 3.40 |
| | Eudragit NE30D | 3.40 | 3.40 | 3.40 | 3.40 | 3.40 |
| | Triethyl citrate | 0.20 | 2.50 | 5.00 | 0.10 | 7.50 |
| | Talc | 1.35 | 1.35 | 1.35 | 1.35 | 1.35 |
| | Purified water | (22.64) | (22.64) | (22.64) | (22.64) | (22.64) |
| Total (mg) of enteric-coated | | 34.10 | 36.40 | 38.90 | 34.00 | 41.40 |
| sustained-release core beads | | | | | | |
| Post mixture | F-Melt Type C | 161.90 | 159.60 | 157.10 | 162.00 | 154.60 |
| | AEROSIL® | 2.00 | 2.00 | 2.00 | 2.00 | 2.00 |
| Lubricant | S-Mg | 2.00 | 2.00 | 2.00 | 2.00 | 2.00 |
| Grand Total (mg) | | 200.00 | 200.00 | 200.00 | 200.00 | 200.00 |

### (2) Analysis of Physical Properties

The density, friability, content uniformity, dissolution, and impurity were tested for the tablets prepared with the compositions of the examples and the comparative examples, and the results are shown.

### <Measurement of Apparent Density and Results thereof>

The enteric-coated core beads of each of the examples and comparative examples and the post mixture were weighed in appropriate amounts, and were put in a 50 mL measuring cylinder, and the volumes were measured. The apparent density of each sample was calculated as mass (mg)/volume (mL). The results are shown in Table 3.

**[Table 3]**

| Apparent density (mg/mL) | Example 1 | Example 2 | Example 3 | Comparative Example 1 | Comparative Example 2 |
|---|---|---|---|---|---|
| Enteric-coated sustained-release core bead A | 0.71 | 0.72 | 0.70 | 0.73 | 0.70 |
| Post mixture B | 0.49 | 0.49 | 0.49 | 0.49 | 0.48 |
| Density ratic (A:B) | 1:0.69 | 1:0.68 | 1:0.7 | 1:0.67 | 1:0.7 |

Referring to Table 3, the density of the sustained-release core bead coated with the enteric coating layer was in a range of 0.70 to 0.73 mg/mL, and the density of the post mixture was in a range of 0.48 to 0.49 mg/mL. Both the core bead and the post mixture exhibited densities within desirable ranges.

### <Friability Test and Test Result>

20 tablets prepared according to the examples and the comparative examples were collected, and the friability evaluation was performed with a friability measuring instrument (device name: TAR200, manufacturer: ERWEKA) at 25 rpm for 4 minutes. The results are shown in Table 4.

**[Table 4]**

| | Example 1 | Example 2 | Example 3 | Comparative Example 1 | Comparative Example 2 |
|---|---|---|---|---|---|
| Friability (%) | 0.05 | 0.03 | 0.02 | 0.04 | 0.02 |
| Standard | < 0.5% | | | | |

The reference level of the friability of the tablet was 0.5% or less, and the tablets according to Examples 1 to 3 and Comparative Examples 1 to 2 satisfied the reference level.

### <Content Uniformity Test and Test Result>

To evaluate the content uniformity of tamsulosin hydrochloride throughout each tablet, it is checked whether the uniformity of dosage unit for each tamsulosin hydrochloride tablet satisfies a reference level of 15 or lower which is specified in the United States Pharmacopoeia (USP).

10 tablets prepared according to in the examples and comparative examples were collected and tested. Each tablet was put in a 50 mL flask, 10 mL of methanol was added thereto, and ultrasound extraction was performed for 30 minutes. After cooling, the marks of the flasks were aligned with a mobile phase, and the solutions of the flasks were filtered with membrane filters with meshes of 0.45 µm or smaller. The filtrates were taken as samples, and the samples were subjected to high-performance liquid chromatography (HPLC). The HPLC conditions are as follows:
- Analysis conditions
   Columns: 4.6 x 150 mm, 5 µm, ODS or similar columns
   Mobile phase: perchlorinated aqueous solution (adjusted to pH 2.0 with sodium hydroxide): acetonitrile = 5:2
   Detector: Ultraviolet absorption spectrophotometer (wavelength: 225 nm)
   Temperature: Approximately 40°C
   Flow rate: 1.0 mL//min
   Filling volume: 50 µl

In the content uniformity test, the smaller the value, the more uniformly the content of tamsulosin hydrochloride is present in each tablet.

**[Table 5]**

| | Example 1 | Example 2 | Example 3 | Comparative Example 1 | Comparative Example 2 |
|---|---|---|---|---|---|
| Determination value | 3.2 | 4.1 | 2.5 | 3.5 | 3.8 |
| Standard | 15 or less | | | | |

As a result of the content uniformity test, it was confirmed that all of the tablets according to Examples 1 to 3 and Comparative Examples 1 and 2 exhibited very good content uniformity. That is, the values indicating the uniformity level were less than 15 that is a reference level defined in the US Pharmacopoeia.

### <Dissolution Test (Acid Resistance Test) and Results>

Dissolution tests were performed under conditions described below.
- Dissolution test conditions
   1) Dissolution method: US Pharmacopoeia (USP) dissolution test method 2 (paddle method)
   2) Dissolution solution: Artificial gastric fluid 500 ml (pH 1.2)
   3) Volume of dissolution solution: 500 ml
   4) Temperature of dissolution instrument: 37°C ± 0.5°C
   5) Paddle speed: 75 rpm
   6) Number of test groups: 6
   7) Sample Collection and Analysis Method

In 30, 60, and 120 minutes after the start of the test, 10 ml of each dissolution test solution was collected, filtered, and subjected to high performance liquid chromatography (HPLC method) under analysis conditions described below.
- Analysis conditions
- Columns: 4.6 x 150 mm, 5 µm, ODS or similar columns
- Mobile phase: perchlorinated aqueous solution (adjusted to pH 2.0 with sodium hydroxide): acetonitrile = 5:2
- Detector: Ultraviolet absorption spectrophotometer (wavelength: 225 nm)
- Temperature: Approximately 40°C
- Flow rate: 1.0 mL/min
- Filling volume: 400 µl

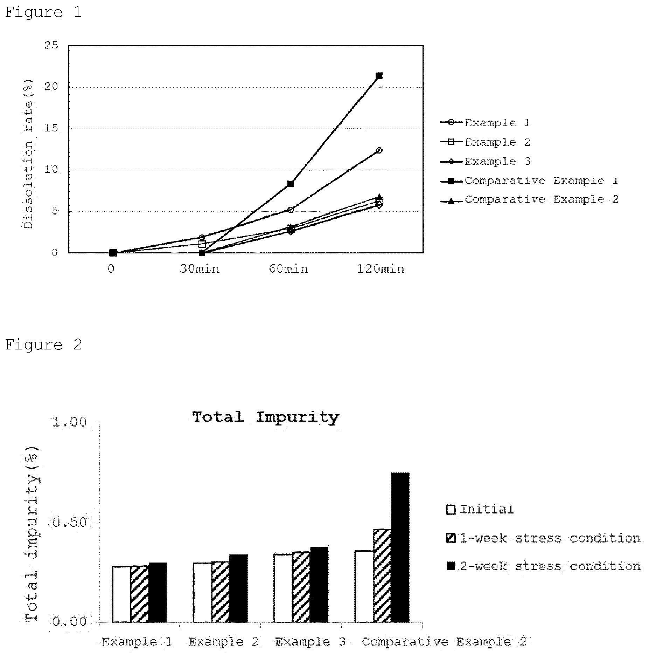

The obtained results are shown in Table 6 and FIG. 1, wherein the numbers in parentheses represent the deviations.

FIG. 1 is a graph showing change in dissolution rate with time for each tablet of the tablets prepared according to Examples 1 to 3 and Comparison examples 1 and 2.

The lower the dissolution rate, the better the acid resistance. It is preferable that the dissolution rate after 2 hours is 20% or less. A higher number in the parentheses represents less uniformity among the products.

**[Table 6]**

| | Dissolution rate over time (deviation) | | | |
|---|---|---|---|---|
| | 0 | 30 minutes | 60 minutes | 120 minutes |
| Example 1 | - | 1.9% (0.1) | 5.2% (0.5) | 12.4% (1.2) |
| Example 2 | - | 1.1% (0.4) | 3.0% (0.3) | 6.3% (0.6) |
| Example 3 | - | 1.2% (0.4) | 2.6% (0.3) | 5.8% (0.6) |
| Comparative Example 1 | - | 3.5% (0.3) | 8.3% (0.5) | 21.4% (1.2) |
| Comparative Example 2 | - | 1.3% (0.2) | 3.2% (0.3) | 6.8% (0.6) |

Referring to Table 6 and FIG. 1, the tablets of Examples 1 to 3 showed a dissolution rate of 20% or less after 2 hours, indicating good acid resistance.

In contrast, the table of Comparison Example 1 exhibited a high initial dissolution rate of 3.5% within 30 minutes. The tablet of Comparative Example 1 has a plasticizer content lower than those of Examples 1 to 3 and Comparative Example 2. In addition, the dissolution rate after 120 minutes was 21.4%, indicating low acid resistance.

In the case of Comparative Example 2, the dissolution rate was as low as 6.8% due to the relatively high content of the plasticizer but was higher than that of Example 3 in which less plasticizer was used. From these results, it is confirmed that the plasticizer has an effect on the acid resistance of the tablet, and the higher the content, the higher the acid resistance. However, when the content is beyond a certain percent, there is a limit to the improvement of the acid resistance.

### <Test of Impurity and Result>

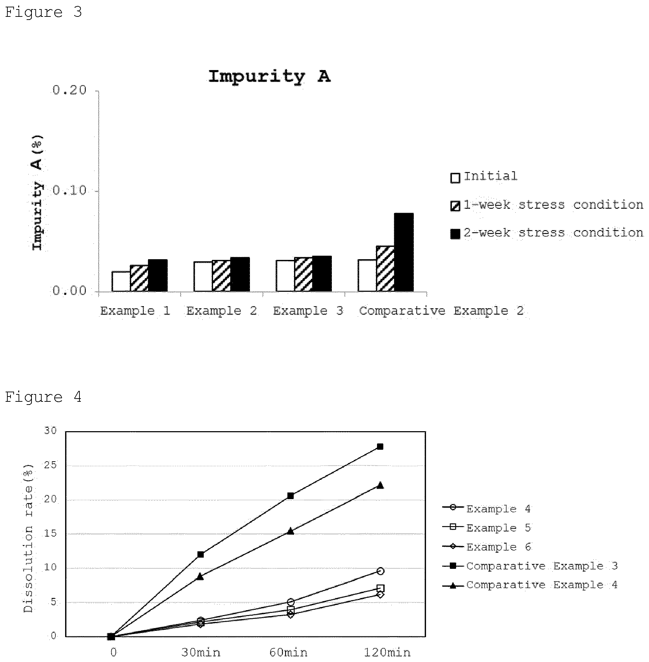

In order to evaluate the stability of tamsulosin according to a change in the content of a plasticizer, 10 tablets prepared in each of the examples and comparative examples were collected, then exposed to stress conditions (for example, 60°C) for 1 week or 2 weeks, and then put into a 50 mL flask. Next, 20 mL of methanol was added thereto, and ultrasonic extraction was performed for 10 minutes. After cooling, the marks of the flasks were aligned with a mobile phase, and the solutions of the flasks were filtered with membrane filters with meshes of 0.45 µm or smaller. The filtrates were taken as samples, and the samples were subjected to high-performance liquid chromatography (HPLC) under conditions described below. The obtained results are presented in FIGS. 2 and 3.
- Analysis conditions
- Columns: 4.6 x 150 mm, 5 µm, ODS or similar columns
- Mobile phase: perchlorinated aqueous solution (adjusted to pH 2.0 with sodium hydroxide): acetonitrile = 7:3
- Detector: Ultraviolet absorption spectrophotometer (wavelength: 225 nm)
- Temperature: Approximately 40°C
- Flow rate: 1.0 mL/min
- Filling volume: 50 µl

FIG. 2 is a graph showing changes in the total content of impurities in each of the tablets prepared according to Examples 1 to 3 and Comparative Examples 1, measured at an initial stage, after undergoing one-week stress conditions, and after undergoing two-week stress conditions.

Referring to FIG. 2, it was confirmed that the tablets of Examples 1 to 3 generated insignificant amounts of impurities even under 2-week stress conditions. It means that significant decomposition of tamsulosin hydrochloride present in the core beads does not occur under 2-week stress conditions. In contrast, it was confirmed that in the case of the tablet of Comparative Example 2, the total amount of impurities significantly increased under stress conditions.

In relation to the impurities, a change in the content of an impurity A was observed.

FIG. 3 is a graph showing changes in the content of an impurity A for each of the tablets prepared according to Examples 1 to 3 and Comparative Examples 1, measured at an initial stage, after undergoing one-week stress conditions, and after undergoing two-week stress conditions. A substance of which a relative retention time (RRT) with respect to a retention time of a main ingredient is 0.354 is referred to as the impurity A. In this case, the impurity A means decomposition products of tamsulosin hydrochloride, and the higher the value, the more severely the decomposition of tamsulosin hydrochloride occurs.

Referring to FIG. 3, in the case of the tablet of Comparative Example 2, the content of the impurity A significantly increases under stress conditions. That is, in the case of the tablet of Comparative Example 2 in which the plasticizer was excessively used, it is confirmed from the results of FIG. 3 that the decomposition of the tamsulosin hydrochloride occurs so that the stability of tamsulosin hydrochloride is deteriorated.

These results show that the use of a plasticizer improves the acid resistance, but when used beyond a certain range, the stability of tamsulosin hydrochloride is adversely affected. Therefore, it is desirable to limit the content of the plasticizer in a tablet.

### Experimental Example 2: Comparison of Physical Properties between Tablets on Content of Buffer in Post mixture

The post mixture of the tablet is mixed with the enteric-coated core beads so that the post mixture portion is formed through the tableting process. During the tableting process, damage to the enteric coating layer occurs, resulting in a decrease in acid resistance and damage to the tablet. That is, quality degradation occurs during the tableting process. Accordingly, the density of the post mixture portion is adjusted so that the content of the buffer in the post mixture portion will fall within a predetermined range. In this case, it is possible to improve the quality of tablets and to increase the acid resistance of the tablets. In this experimental example, each tablet was manufactured by varying the content of the plasticizer in the post mixture portion, and the physical properties of the prepared tablets were compared. As the buffer, colloidal silica (AEROSIL®) was used.

### -(1) Preparation of Tablet

Tablets were prepared in accordance with the compositions shown in Table 7. As core beads, the sustained-release core beads prepared according to Preparation Example 1 were used.

Eudragit L30-D55 and Eudragit NE30D were used as an enteric coating material, and triethyl citrate was used as the plasticizer. Eudragit L30-D55 and Eudragit NE30D were dissolved in purified water in a mixer, talc and triethyl citrate were added thereto to prepare an enteric coating solution.

The sustained-release core beads prepared in Preparation Example 1 were collected and put into a fluidized bed system, and coating was carried by spraying the prepared coating solution from the bottom. After the spraying of the coating solution was finished, microspheres (i.e., enteric-coated sustained-release core beads) that are the sustained-release core beads on the surface of which an enteric coating layer is formed were obtained.

Tablets were prepared by adding a post mixture including F-Melt Type C, AEROSIL®, and a lubricant to the microspheres and by tableting the resultant. In Examples 4 to 6, the contents of AEROSIL® used as the buffer was 0.5% by weight, 1.5% by weight, and 2.5% by weight, respectively. In Comparative Examples 4 to 6, the contents were adjusted to 0.25% by weight, 3.5% by weight, and 4.5% by weight, respectively.

**[Table 7]**

| Composition | | Example 4 | Example 5 | Example 6 | Comparative Example 3 | Comparative Example 4 | Comparative Example 5 | Comparative Example 6 |
|---|---|---|---|---|---|---|---|---|
| Sustained-release core beads (mg) | | 25.75 | 25.75 | 25.75 | 25.75 | 25.75 | 25.75 | 25.75 |
| Enteric coating solution | Eudragit L3 | 3.40 | 3.40 | 3.40 | 3.40 | 3.40 | 3.40 | 3.40 |
| | Eudragit NE30D | 3.40 | 3.40 | 3.40 | 3.40 | 3.40 | 3.40 | 3.40 |
| | Triethyl citrate | 2.50 | 2.50 | 2.50 | 2.50 | 2.50 | 2.50 | 2.50 |
| | Talc | 1.35 | 1.35 | 1.35 | 1.35 | 1.35 | 1.35 | 1.35 |
| | Purified water | (22.64) | (22.64) | (22.64) | (22.64) | (22.64) | (22.64) | (22.64) |
| Total (mg) of coated sustained-release core beads | | 36.40 | 36.40 | 36.40 | 36.40 | 36.40 | 36.40 | 36.40 |
| Post mixture | F-Melt Type C | 160.6 | 158.6 | 156.6 | 161.6 | 161.1 | 154.6 | 152.6 |
| | Aerosil | 1.00 | 3.00 | 5.00 | - | 0.50 | 7.00 | 9.00 |
| Lubricant | S-Mg | 2.00 | 2.00 | 2.00 | 2.00 | 2.00 | 2.00 | 2.00 |
| Grand Total (mg) | | 200.00 | 200.00 | 200.00 | 200.00 | 200.00 | 200.00 | 200.00 |

### (2) Analysis of Physical Properties

The density, friability, content uniformity, and dissolution were tested for the tablets prepared with the compositions of the examples and the comparative examples using the same test method used in Experimental Example 1, and the results are shown.

### <Measurement of Apparent Density and Results>

**[Table 8]**

| Apparent density (mg/mL) | Example 4 | Example 5 | Example 6 | Comparative Example 3 | Comparative Example 4 | Comparative Example 5 | Comparative Example 6 |
|---|---|---|---|---|---|---|---|
| Enteric-coated sustained-release core bead A | 0.72 | 0.72 | 0.72 | 0.72 | 0.72 | 0.72 | 0.72 |
| Post mixture B | 0.51 | 0.46 | 0.42 | 0.55 | 0.53 | 0.37 | 0.32 |
| Density ratio (A:B) | 1:0.71 | 1:0.64 | 1:0.58 | 1:0.76 | 1:0.74 | 1:0.51 | 1:0.44 |

Referring to Table 8, the density of the post mixture portion varies within a range of 0.32 to 0.55 mg/mL, which is due to the difference in the content of the buffer (i.e., AEROSIL®) included in the post mixture portion.

Specifically, Comparative Example 3 was a tablet without a buffer, and the density of the post mixture portion was 0.55 mg/mL. When the buffer was included as in Examples 4 and 6 and Comparative Examples 4 and 6, as the content of the buffer was increased, the density was gradually decreased.

In the tablets of Examples 4 to 6, the densities of the post mixture portions were 0.51 mg/mL, 0.46 mg/mL, and 0.42 mg/mL, respectively, satisfying the range of 0.40 to 0.52 mg/mL suggested by the present invention. However, in the tablets of Comparative Examples 5 and 6 included 3.5% by weight or more of the buffer, the densities of the post mixture portions were 0.37 mg/mL and 0.32 mg/mL, respectively, showing values less than 0.4 mg/mL. From these results, it is seen that the content of the buffer directly affects the density of the post mixture portion. This density range is directly related to the friability, content uniformity and acid resistance presented below, and improves the physical properties.

### <Friability Test and Test Result>

**[Table 9]**

| | Example 4 | Example 5 | Example 6 | Comparative Example 3 | Comparative Example 4 | Comparative Example 5 | Comparative Example 6 |
|---|---|---|---|---|---|---|---|
| Friability (%) | 0.06 | 0.21 | 0.39 | 0.01 | 0.03 | 0.93 | 1.49 |
| Standard | 0.5% or less | | | | | | |

For Examples 4 to 6 and Comparative Examples 3 to 6, the standard of the friability of the tablet in Experimental Example 1 was 0.5% or less. Referring to Table 9, the tablets of Examples 4 to 6 and Comparative Examples 3 and 4 satisfied the specified range, but the tablets of Comparative Examples 5 and 6 did not satisfied the range. Thus, the tablets of Comparative Examples 5 and 6 were significantly damaged.

These results, as shown in Table 8, indicate that the content of the buffer is directly related to the friability. The tablet of Comparison Example 3 showed the lowest friability because it did not contain the buffer. In addition, as the content of the buffer is increased as in Example 4, 5, 6, and Comparison Example 4, the friability was increased. However, the tablets of Comparative Examples 5 and 6 in which the buffer was excessively used (i.e., the content is higher than 3.5% by weight) exhibited significant loss. For example, the largest loss was three times higher than a standard level of 0.5%. From these results, it is seen that the content of the buffer needs to be controlled.

### <Content Uniformity Test and Test Result>

**[Table 10]**

| | Example 4 | Example 5 | Example 6 | Comparative Example 3 | Comparative Example 4 | Comparative Example 5 | Comparative Example 6 |
|---|---|---|---|---|---|---|---|
| Determination value | 4.5 | 6.7 | 9.2 | 4.1 | 3.9 | 15.4 | 21.2 |
| Standard | 15 or less | | | | | | |

The content uniformity was defined in the US Pharmacopoeia 15 or less. It was confirmed that the tablets of Examples 4 to 6 and Comparative Examples 3 and 4 were all at an appropriate level and had very excellent content uniformity among dosage units.

In contrast, the tablets of Comparative Examples 5 and 6 exhibited the uniformity levels deviated from the standard level so that the tablets were determined inappropriate. These results are directly related to the content of the buffer. Since the content uniformity tends to decrease as the content of the buffer increases, it is seen that it is necessary to control the content of the buffer.

### <Dissolution Test (Acid Resistance Test) and Results>

When the content of the buffer was excessive, as shown in Tables 8, 9, and 10, the density, friability, and content uniformity were decreased. In the case of Comparative Examples 3 and 4, good physical properties were exhibited when the buffer was not included or contained little, but the acid resistance of the tablets were decreased. In the present experimental example, the acid resistances of the tablets were compared for the tablet of Comparative Example 3 in which no buffer is contained, the tablet of Comparative Example 4 in the buffer is contained little, and the tablets of Examples 4 to 6. From the results, the lower limit of an optimum content range of the buffer was estimated.

FIG. 4 is a graph showing changes in dissolution rate with time for each of the tablets prepared according to Examples 4 to 6 and Comparative Examples 3 and 4.

The lower the dissolution rate, the better the acid resistance. It is preferable that the dissolution rate after 2 hours is 20% or less. A higher number in the parentheses represents less uniformity among the products.

**[Table 11]**

| | Dissolution rate over time (deviation) | | | |
|---|---|---|---|---|
| | 0 | 30 minutes | 60 minutes | 120 minutes |
| Example 4 | - | 2.4% (0.1) | 5.1% (0.6) | 9.6% (0.5) |
| Example 5 | - | 2.1% (0.3) | 3.9% (0.4) | 7.1% (0.2) |
| Example 6 | - | 1.8% (0.5) | 3.2% (0.9) | 6.2% (0.8) |
| Comparative Example 3 | - | 12.0% (0.8) | 20.6% (1.0) | 27.8% (2.1) |
| Comparative Example 4 | - | 8.8% (0.4) | 15.4% (0.8) | 22.1% (3.3) |

Referring to Table 11 and FIG. 4, it is seen that the tablets of Examples 4 to 6 show a dissolution rate of 20% or less after 2 hours, indicating excellent acid resistance.

In contrast, in the case of the tablet of Comparative Example 3 containing no buffer, the initial dissolution rate was very high (12%), and after 120 minutes, the dissolution rate also was very high (27.8%) . That is, the acid resistance of the tablet was low, and the deviation was 2.1 which indicates poor uniformity of unit dosage among products. In addition, as in Comparative Example 4, when the buffer is included in a small amount (i.e., 0.25% by weight), the initial dissolution rate and the dissolution rate after 120 minutes were slightly lowered, but those figures do not satisfy the standard level.

From these results, it is confirmed that a buffer has an effect on the acid resistance of tablets. The higher the content, the higher the acid resistance. However, when the content of a buffer is beyond a certain percent, there is a limit to the improvement of the acid resistance.

### Experimental Example 3: Comparison of Physical Properties between Tablets on Type of Disintegrant in Post mixture

The post mixture portion of each tablet includes a predetermined amount of buffer to improve acid resistance. The post mixture portion includes a disintegrant as the balance as with the case of a buffer. In this case, the type of the disintegrant affects the acid resistance of the tablet as well as the density, friability, and content uniformity of the post mixture portion. In this experimental example, each tablet was manufactured by varying the type of disintegrant, and the physical properties of the prepared tablets were compared. As the disintegrant, F-Melt Type C, Mannitol, Pregelatinized Starch, and lactose were used.

### (1) Preparation of Tablet

Tablets were prepared in accordance with the compositions shown in Table 12. As core beads, the sustained-release core beads prepared according to Preparation Example 1 were used.

Eudragit L30-D55 and Eudragit NE30D were used as an enteric coating material, and triethyl citrate was used as a plasticizer. Eudragit L30-D55 and Eudragit NE30D were dissolved in purified water in a mixer, talc and triethyl citrate were added thereto to prepare an enteric coating solution.

The sustained-release core beads prepared in Preparation Example 1 were collected and put into a fluidized bed system, and coating was carried by spraying the prepared coating solution from the bottom. After the spraying of the coating solution was finished, microspheres sustained-release core beads that are core beads on the surface of which an enteric coating layer is formed were obtained.

Tablets were prepared by adding a post mixture including F-Melt Type C, AEROSIL®, and a lubricant to the microspheres and by tableting the resultant.

**[Table 12]**

| Composition | | Example 7 | Example 8 | Example 9 | Example 10 |
|---|---|---|---|---|---|
| Sustained-release core beads (mg) | | 25.75 | 25.75 | 25.75 | 25.75 |
| Enteric coating solution | Eudragit L3 | 3.40 | 3.40 | 3.40 | 3.40 |
| | Eudragit NE30D | 3.40 | 3.40 | 3.40 | 3.40 |
| | Triethyl citrate | 2.50 | 2.50 | 2.50 | 2.50 |
| | Talc | 1.35 | 1.35 | 1.35 | 1.35 |
| | Purified water | (22.64) | (22.64) | (22.64) | (22.64) |
| Total (mg) of enteric-coated sustained-release core beads | | 36.40 | 36.40 | 36.40 | 36.40 |
| Post mixture | F-Melt Type C | 157.6 | - | - | - |
| | Mannitol | - | 157.6 | - | - |
| | Pregelatinzed starch | - | - | 157.6 | - |
| | lactose | - | - | - | 157.6 |
| | Aerosil | 4.00 | 4.00 | 4.00 | 4.00 |
| Lubricant | S-Mg | 2.00 | 2.00 | 2.00 | 2.00 |
| Grand Total (mg) | | 200.00 | 200.00 | 200.00 | 200.00 |

### (2) Analysis of Physical Properties

The density, friability, content uniformity, and dissolution were measured using the tablets according to the examples, and the results are shown. Each test was conducted in the same manner as described in Experimental Example 1.

### <Measurement of Apparent Density and Results>

**[Table 13]**

| Apparent density (mg/mL) | Example 7 | Example 8 | Example 9 | Example 10 |
|---|---|---|---|---|
| Enteric-coated sustained-release core bead (A) | 0.72 | 0.72 | 0.72 | 0.72 |
| Post mixture (B) | 0.44 | 0.41 | 0.50 | 0.51 |
| Density ratio (A:B) | 1: 0.61 | 1:0.57 | 1:0.69 | 1:0.71 |

Referring to Table 13 above, the densities of the post mixture portions of the tablets were in a range of 0.40 to 0.52 mg/mL, thereby satisfying an appropriate density range required for tablets.

### <Friability Test and Test Result>

**[Table 14]**

| | Example 7 | Example 8 | Example 9 | Example 10 |
|---|---|---|---|---|
| Friability (%) | 0.29 | 0.31 | 0.30 | 0.30 |
| Standard | 0.5% or less | | | |

The reference level of the friability of tablets was set to be 0.5% or less. Referring to Table 14, the tablets according to Examples 7 to 10 satisfied the reference level.

### <Content Uniformity Test and Test Result>

**[Table 15]**

| | Example 7 | Example 8 | Example 9 | Example 10 |
|---|---|---|---|---|
| Determination value | 6.9 | 7.1 | 7.3 | 7.0 |
| Standard | 15 or less | | | |

The content uniformity was 15 or less, thereby satisfying the standard level prescribed in the US Pharmacopoeia. It is confirmed that all of the tablets of Examples 7 to 10 have very excellent content uniformity among dosage units.

### <Dissolution Test (Acid Resistance Test) and Results>

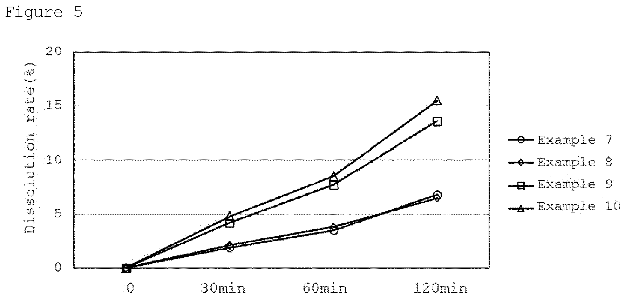

The disintegrant belongs to the post mixture portion. Thus, a test was performed to determine whether the type of disintegrant affects acid resistance. The test results are shown in Table 16 and FIG. 5.

FIG. 5 is a graph showing changes in dissolution rate with time for each of the tablets prepared according to Examples 7 to and 10.

The lower the dissolution rate, the better the acid resistance. It is preferable that the dissolution rate after 2 hours is 20% or less. A higher number in the parentheses represents less uniformity among dosage units.

**[Table 16]**

| | Dissolution rate over time (deviation) | | | |
|---|---|---|---|---|
| | 0 | 30 minutes | 60 minutes | 120 minutes |
| Example 7 | - | 1.9% (0.1) | 3.5% (0.5) | 6.8% (0.8) |
| Example 8 | - | 2.1% (0.7) | 3.8% (1.2) | 6.5% (0.9) |
| Example 9 | - | 4.2% (0.3) | 7.7% (0.6) | 13.6% (1.8) |
| Example 10 | - | 4.8% (0.5) | 8.5% (1.1) | 15.5% (2.4) |

Referring to Table 16 and FIG. 5, when F-Melt Type C and Mannitol were used as a disintegrant, the dissolution rates after 120 minutes 10% or less, indicating good acid resistance. In addition, the deviation was 1.0 or less, indicating good uniformity among dosage units. In addition, it is that in the case of using pregelatinized starch and lactose, the dissolution rate was 20% or less, which exhibits excellent acid resistance. Therefore, pregelatinized starch and lactose can be suitably used as the disintegrant.

From these results, it is seen that it is possible to easily control the acid resistance by appropriately selecting the type of disintegrant.

### Experimental Example 4: Comparison of Physical Properties between Tablets on Content of Lubricant

### (1) Preparation of Tablet

Tablets were prepared in accordance with the composition shown in Table 17. As core beads, the sustained-release core beads prepared according to Preparation Example 1 were used.

Eudragit L30-D55 and Eudragit NE30D were used as an enteric coating material, and triethyl citrate was used as the plasticizer. Eudragit L30-D55 and Eudragit NE30D were dissolved in purified water in a mixer, talc and triethyl citrate were added thereto to prepare an enteric coating solution.

The sustained-release core beads prepared in Preparation Example 1 were collected and put into a fluidized bed system, and coating was carried by spraying the prepared coating solution from the bottom. After the spraying of the coating solution was finished, microspheres sustained-release core beads) that are core beads on the surface of which an enteric coating layer is formed were obtained.

Tablets were prepared by adding a post mixture including disintegrant, AEROSIL®, and lubricant to the microspheres and by tableting the resultant. The tablets were manufactured by varying the type of disintegrant and the content of lubricant, as shown in Table 17.

**[Table 17]**

| Composition | Example 7 | Example 11 | Comparative Example 7 | |
|---|---|---|---|---|
| Sustained-release core beads | 25.75 | 25.75 | 25.75 | |
| (mg) | | | | |
| Enteric coating solution | Eudragit L3 | 3.40 | 3.40 | 3.40 |
| | Eudragit NE30D | 3.40 | 3.40 | 3.40 |
| | Triethyl citrate | 2.50 | 2.50 | 2.50 |
| | Talc | 1.35 | 1.35 | 1.35 |
| | Purified water | (22.64) | (22.64) | (22.64) |
| Total (mg) of enteric-coated sustained-release core beads | | 36.40 | 36.40 | 36.40 |
| Post mixture | F-Melt Type C | 157.6 | 155.6 | 159.6 |
| | Aerosil | 4.00 | 4.00 | 4.00 |
| Lubricant | S-Mg | 2.00 | 4.00 | - |
| Grand Total (mg) | | 200.00 | 200.00 | 200.00 |

### (2) Analysis of Physical Properties

Using the tablets of the examples and comparison examples, a test was performed to determine a dissolution rate and to determine whether or not sticking occurred. The results were provided.

### <Sticking Checking Results>

100 tablets according to each of the examples and comparative example were prepared. The number of tablets that exhibited sticking was counted and shown in Table 18.

**[Table 18]**

| | Example 7 | Example 11 | Comparative Example 7 |
|---|---|---|---|
| Sticking | 0 | 0 | 13 |

Referring to Table 18, the tablets in Examples 7 and 11 in which S-Mg was used as a lubricant no sticking during tableting, and 13 out of 100 tablets without S-Mg exhibited sticking. These results show that it is preferable to use a lubricant such as S-Mg during tableting to lower a product defect rate.

### <Dissolution Test (pH 6.8) and Results>

Dissolution tests were performed under conditions described below.
- Dissolution test conditions
   1) Dissolution method: US Pharmacopoeia (USP) dissolution test method 2 (paddle method)
   2) Dissolution solution: Artificial gastric fluid 500 ml (pH 6.8)
   3) Volume of dissolution solution: 500 ml
   4) Temperature of dissolution instrument: 37°C ± 0.5°C
   5) Paddle speed: 75 rpm
   6) Number of test groups: 6
   7) Sample Collection and Analysis Method

In 30 minutes and 60 minutes after the start of the test, 10 ml of each dissolution test solution was collected, filtered, and subjected to high performance liquid chromatography (HPLC method) under analysis conditions described below.
- Analysis conditions
- Columns: 4.6 x 150 mm, 5 µm, ODS or similar columns
- Mobile phase: perchlorinated aqueous solution (adjusted to pH 2.0 with sodium hydroxide): acetonitrile = 5:2
- Detector: Ultraviolet absorption spectrophotometer (wavelength: 225 nm)
- Temperature: Approximately 40°C
- Flow rate: 1.0 mL/min
- Filling volume: 400 µl

The obtained results are shown in Table 19, wherein the numbers in parentheses represent the deviations.

**[Table 19]**

| | Dissolution rate over time (deviation) | | |
|---|---|---|---|
| | 0 | 30 minutes | 60 minutes |
| Standard | - | 20% to 50% | 35% to 65% |
| Example 7 | - | 32.9% (2.8) | 47.2% (3.2) |
| Example 11 | - | 25.2% (2.2) | 42.3% (2.6) |
| Comparative Example 7 | - | 39.1% (2.7) | 52.2% (3.1) |

Referring to Table 19, it is confirmed the tablets of Examples 7, 11, and 7 in which the S-Mg content is in a range of 0.0% to 2.0% by weight satisfies the standard dissolution rates at in 30 minutes and 60 minutes under pH 6.7.

From the results, it is desirable that the content of S-Mg as a lubricant is less than 3% by weight to satisfy the standard dissolution rate.

Referring to Tables 18 to 19, it is confirmed that the suitable content range of S-Mg, which does not interfere with tableting and exhibits good dissolution rate at pH 6.8 is less than 3% by weight, preferably 0.5% to 2.5% by weight, and more preferably 1.0% to 2.0% by weight.

## Claims

1. A pharmaceutical composition containing tamsulosin hydrochloride comprising: tamsulosin hydrochloride as an active ingredient; an enteric polymer; and a plasticizer, wherein the plasticizer is included in a content of 0.10% by weight to 3.5% by weight, based on the total amount of the pharmaceutical composition.

2. The pharmaceutical composition according to claim 1, wherein the plasticizer is at least one or more substances selected from the group consisting of polyethylene glycol, polypropylene glycol, polyglycolic acid, poly(butylene adipate), glycerin, tributyl sebacate, triacetin, and triethyl citrate.

3. The pharmaceutical composition according to claim 1, wherein the enteric polymer is at least one or more acid-resistant acrylic polymer selected from the group consisting of a methacrylic acid-methyl methacrylate copolymer and a methacrylic acid-ethyl acrylate copolymer.

4. The pharmaceutical composition according to claim 1, wherein the pharmaceutical composition has a dissolution rate of 20% or lower after 2 hours when the dissolution rate is measured with a pH 1.2 buffer solution of 500 mL at a rotation speed of 75 rpm according to the USP dissolution test method 2 (paddle method).

5. The pharmaceutical composition according to claim 1, further comprising: a sustained-releasing agent.

6. The pharmaceutical composition according to claim 5, wherein the sustained-releasing agent is at least one or more selected from the group consisting of polyvinyl acetate, a mixture including polyvinyl acetate, ethylcellulose, and hydroxypropylmethylcellulose (HPMC).

7. The pharmaceutical composition according to claim 1, further comprising: a disintegrant, a buffer, or both.

8. The pharmaceutical composition according to claim 7, wherein the buffer is colloidal silicon dioxide, and comprising a content of 0.50% by weight to 3.0% by weight based on the total amount of the pharmaceutical composition.

9. The pharmaceutical composition according to claim 1, further comprising: a lubricant.

10. The pharmaceutical composition according to claim 9, wherein the lubricant is magnesium stearate has a content of less than 3% by weight based on the total amount of pharmaceutical composition.

11. The pharmaceutical composition according to claim 1, wherein the composition is formulated into tablets by coating sustained-release core beads including tamsulosin hydrochloride and a sustained-releasing agent with an enteric coating solution including an enteric polymer and a plasticizer, after mixing the coated core beads with a post mixture including a disintegrant and a buffer, and tableting the obtained mixture.

12. A method of preparing a pharmaceutical composition containing tamsulosin hydrochloride is composed of:
preparing core beads comprising tamsulosin hydrochloride;
preparing sustained-release core beads by coating the core bead with a sustained-release coating solution including a sustained-releasing agent;
preparing enteric-coated sustained-release core beads having an enteric coating layer by coating the sustained-release core beads with an enteric coating solution including an enteric polymer and a plasticizer;
preparing a post mixture including a disintegrant and a buffer;
after mixing the enteric-coated sustained-release core beads, the post mixture, and a lubricant, and tableting the obtained mixture.

13. The method according to claim 12, wherein the preparing of the sustained-release core beads is comprised of preparing core beads including tamsulosin hydrochloride, a binder, and a diluent, and then coating the core beads with a sustained-release coating solution including a sustained-releasing agent.

14. The method according to claim 12, wherein the plasticizer is at least one or more substances selected from the group consisting of polyethylene glycol, polypropylene glycol, polyglycolic acid, poly(butylene adipate), glycerin, tributyl sebacate, triacetin, and triethyl citrate.

15. The method according to claim 12, wherein the post mixture has an apparent density of 0.40 mg/mL to 0.52 mg/mL.

16. The method according to claim 12, wherein the pharmaceutical composition is formulated into tablets.
